(19) Europäisches Patentamt
European Patent Office
Office européen des brevets

(11) EP 1 043 306 B1

(12) EUROPÄISCHE PATENTSCHRIFT

(45) Veröffentlichungstag und Bekanntmachung des Hinweises auf die Patenterteilung:
**31.03.2004 Patentblatt 2004/14**

(21) Anmeldenummer: **00104740.6**

(22) Anmeldetag: **04.03.2000**

(51) Int Cl.7: **C07C 217/52**, C07C 219/24, A61K 31/235, A61K 31/137, A61P 29/00, A61P 23/00, A61P 17/00, A61P 21/00, A61P 25/00, A61P 9/00, A61P 1/00

(54) **3-Amino-3-arylpropan-1-ol-Derivate, deren Herstellung und Verwendung**

3-amino-3-arylpropan-1-ol-derivates, their preparation and use

Dérivés 3-Amino-3-arylpropan-1-ol leur préparation et leur usage

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **07.04.1999 DE 19915602**

(43) Veröffentlichungstag der Anmeldung:
**11.10.2000 Patentblatt 2000/41**

(73) Patentinhaber: **Grünenthal GmbH**
**52078 Aachen (DE)**

(72) Erfinder:
- **Sundermann, Bernd, Dr.**
**52072 Aachen (DE)**
- **Kögel, Babette-Yvonne, Dr.**
**52379 Langerwehe-Hamich (DE)**
- **Hennies, Hagen-Heinrich, Dr.**
**52152 Simmerath (DE)**
- **Buschmann, Helmut, Dr.**
**52066 Aachen (DE)**

(56) Entgegenhaltungen:
**EP-A- 1 043 307**
**US-A- 3 937 818**
**US-A- 3 975 436**
**US-A- 4 143 158**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 043 306 B1

## Beschreibung

Die Erfindung betrifft 3-Amino-3-arylpropan-1-ol-Derivate, der allgemeinen Formel I,

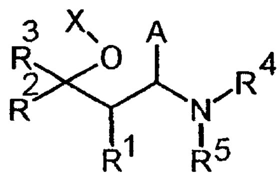

I

worin

$R^1$, $R^2$ ,jeweils unabhängig voneinander, $C_{1-6}$-Alkyl oder $R^1$ und $R^2$ zusammen einen $(CH_2)_{2-6}$-Ring bilden, der auch benzokondensiert oder phenylsubstituiert sein kann

$R^3$ H, Methyl

$R^4$, $R^5$ ,jeweils unabhängig voneinander, $C_{1-6}$-Alkyl, $C_3$-$C_6$-Cycloalkyl, Phenyl, Benzyl, Phenethyl oder $R^4$ und $R^5$ zusammen einen $(CH_2)_{3-6}$- oder $CH_2CH_2OCH_2CH_2$-Ring bilden

A einen Rest aus der Gruppe von substituiertem Phenyl der Formel XIII

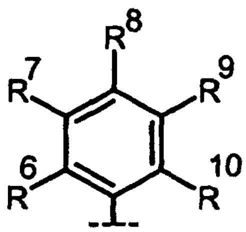

XIII

oder einen einfach oder mehrfach mit $R^6$ bis $R^{10}$ substituierten Furanyl-, Thiophenyl-, Pyrrolyl-, Pyridinyl-, Pyrimidinyl-, Chinolinyl-, Isochinolinyl-, Phtalazinyl- oder Chinazolinyl-Rest darstellt, wobei

$R^6$ bis $R^{10}$ ,jeweils unabhängig voneinander, H, F, Cl, Br, I, $CF_3$, OH, $OR^{11}$, $OCF_3$, $SR^{11}$, $SO_2CH_3$, $SO_2CF_3$, $C_{1-6}$-Alkyl, Phenyl, CN, $COOR^{11}$, $NO_2$ bedeuten oder $R^6$ und $R^7$ oder $R^7$ und $R^8$ zusammen einen $OCH_2O$- oder $OCH_2CH_2O$-Ring bilden,

X ein substituiertes Benzyl der Formel XI

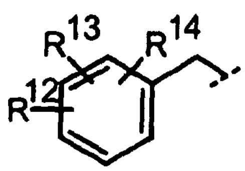

XI

oder ein substituiertes Benzoyl der Formel XII

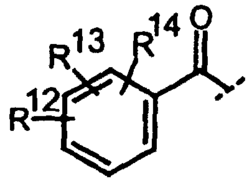

XII,

worin

$R^{12}$ bis $R^{14}$ jeweils unabhängig voneinander, H, F, Cl, Br, $CHF_2$, $CF_3$, $OR^{11}$, $SR^{11}$, $OCF_3$, $SO_2CH_3$, $SO_2CF_3$, $C_{1-6}$-Alkyl, Phenyl, CN, $COOR^{11}$, $NO_2$ mit

$R^{11}$ H, $C_{1-6}$-Alkyl, Phenyl, Benzyl, Phenethyl

bedeuten,
und deren Diastereomere oder Enantiomere in Form ihrer Basen oder Salze physiologisch verträglicher Säuren, sowie deren Herstellung und Verwendung als Arzneimittel.

Die Behandlung chronischer und nichtchronischer Schmerzzustände hat in der Medizin eine große Bedeutung, denn Schmerz gehört zu den Basissymptomen in der Klinik. Zur Zeit besteht ein weltweiter Bedarf an zusätzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie z.B. Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation, Sucht, Abhängigkeit und Toleranzentwicklung limitiert. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990). Außerdem sind sie bei einigen Schmerzzuständen, insbesondere bei neuropathischen Schmerzen, weniger wirksam.

Es sind bereits 3-Amino-3-arylpropan-1-ol-Derivate in der Literatur beschrieben:

US 4 143 158 beschreibt (1)-α-Dimethylamino-α-(cis-2-benzyloxycyclohexyl)-m-cresol zur Inhibierung der Prolactin-Freisetzung.

US 3 975 436 beschreibt α-Dimethylamino-α-(cis-2-benzyloxycyclohexyl)-m-cresol zur Antagonisierung der pharmakologischen Effekte von Narkotika.

In US 3 937 818 sind die Verbindungen α-Dimethylamino-α-(cis-2-benzyloxycyclohexyl)-m-cresol und α-Dimethylamino-α-(cis-2-ethoxycyclohexyl)-m-cresol zur Antagonisierung der Effekte eines Narkotikums beschrieben.

Die der Erfindung zugrundeliegende Aufgabe bestand darin, eine neue Strukturklasse analgetisch wirksamer Substanzen zu finden, die sich zur Schmerztherapie eignen. Weitere Aufgaben bestanden darin, Wirkstoffe zu finden, die sich auch zur Verwendung als Lokalanästetikum und/oder Antiarrhythmikum und/oder Antiemetikum und/oder Nootropikum (Neurotropikum) und/oder zur Behandlung/Therapie von: cardiovaskulären Erkrankungen und/oder Harninkontinenz und/oder Diarrhöe und/oder Pruritus und/oder Entzündungen eignen.

Es wurde gefunden, daß die Verbindungsklasse der allgemeinen Formel I sich durch eine ausgeprägte analgetische Wirkung auszeichnet. Darüber hinaus zeigen die Verbindungen, der allgemeinen Formel I deutliche Affinität zur Bindungsstelle 2 des Natriumkanals (BTX-Bindung) und zur Benzothiazepin-Bindungsstelle des L-Typ Kalziumkanals (Diltiazem-Bindung). Dadurch ist die Verbindungsklasse der allgemeinen Formel I auch zur Verwendung als Lokalanästetikum und/oder Antiarrhythmikum und/oder Antiemetikum und/oder Nootropikum (Neurotropikum) und/oder zur Behandlung/Therapie von cardiovaskulären Erkrankungen und/oder Harninkontinenz und/oder Diarrhöe und/oder Pruritus und/oder Entzündungen geeignet.

Die Erfindung betrifft daher 3-Amino-3-arylpropan-1-ol-Derivate der allgemeinen Formel I und deren Diastereomere oder Enantiomere in Form ihrer Basen oder Salze physiologisch verträglicher Säuren.

Bevorzugt sind Verbindungen der allgemeinen Formel I, in denen $R^1$ und $R^2$ zusammen einen $(CH_2)_{2-6}$-Ring

bilden, der auch benzokondensiert oder phenylsubstituiert sein kann, $R^3$ bis $R^5$, A und X die Bedeutung gemäß der Definition der allgemeinen Formel I haben oder
Verbindungen der allgemeinen Formel I, in denen $R^1$ und $R^2$ zusammen einen $(CH_2)_4$-Ring bilden, der auch benzokondensiert oder phenylsubstituiert sein kann, $R^3$ bis $R^5$, A und X die Bedeutung gemäß der Definition der allgemeinen Formel I haben oder
Verbindungen der allgemeinen Formel I, in denen $R^3$ ein Rest aus der Gruppe von Wasserstoff oder Methyl darstellt, $R^1$, $R^2$, $R^4$ bis $R^5$, A und X die Bedeutung gemäß der Definition der allgemeinen Formel I haben oder
Verbindungen der allgemeinen Formel I, in denen A einen Rest aus der Gruppe von substituiertem Phenyl der Formel XIII

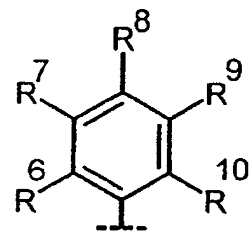

XIII

wobei

| | |
|---|---|
| $R^6$ bis $R^{10}$ | ,jeweils unabhängig voneinander, H, F, Cl, Br, I, $CF_3$, OH, $OR^{11}$, $OCF_3$, $SR^{11}$, $SO_2CH_3$, $SO_2CF_3$, $C_{1-6}$-Alkyl, Phenyl, CN, $COOR^{11}$, $NO_2$ oder $R^6$ und $R^7$ oder $R^7$ und $R^8$ zusammen einen $OCH_2O$- oder $OCH_2CH_2O$-Ring bilden, |
| $R^{11}$ | $C_{1-6}$-Alkyl, Phenyl, Benzyl, Phenethyl |

oder Thiophen oder Furan bedeutet und $R^1$ bis $R^5$ und X die Bedeutung gemäß der Definition der allgemeinen Formel I haben oder
Verbindungen der allgemeinen Formel I, in der $R^1$ und $R^2$ zusammen einen $(CH_2)_{2-6}$-Ring bilden, der auch benzokondensiert oder phenylsubstituiert sein kann, $R^3$ ein Rest aus der Gruppe von Wasserstoff oder Methyl darstellt, $R^4$ bis $R^5$, A und X die Bedeutung gemäß der Definition der allgemeinen Formel I haben oder
Verbindungen der allgemeinen Formel I, in der $R^1$ und $R^2$ zusammen einen $(CH_2)_4$-Ring bilden, der auch benzokondensiert oder phenylsubstituiert sein kann, A einen Rest aus der Gruppe von substituiertem Phenyl der Formel XIII oder Thiophen oder Furan, $R^3$ ein Rest aus der Gruppe von Wasserstoff oder Methyl darstellt, $R^4$ bis $R^5$ und X die Bedeutung gemäß der Definition der allgemeinen Formel I haben oder
Verbindungen der allgemeinen Formel I, in der $R^1$ und $R^2$ zusammen einen $(CH_2)_4$-Ring bilden, A einen Rest aus der Gruppe von substituiertem Phenyl der Formel XIII oder Thiophen oder Furan, $R^3$ ein Rest aus der Gruppe von Wasserstoff oder Methyl darstellt, $R^4$ bis $R^5$ und X die Bedeutung gemäß der Definition der allgemeinen Formel I haben oder
Verbindungen der allgemeinen Formel I, in der $R^1$ und $R^2$ zusammen einen $(CH_2)_4$-Ring bilden, A Thiophen, $R^3$ ein Rest aus der Gruppe von Wasserstoff oder Methyl darstellt, $R^4$ bis $R^5$ und X die Bedeutung gemäß der Definition der allgemeinen Formel I haben oder
Verbindungen der allgemeinen Formel I, in der $R^1$ und $R^2$ zusammen einen $(CH_2)_4$-Ring bilden, A Furan, $R^3$ ein Rest aus der Gruppe von Wasserstoff oder Methyl darstellt, $R^4$ bis $R^5$ und X die Bedeutung gemäß der Definition der allgemeinen Formel I haben oder
Verbindungen der allgemeinen Formel I, in der X einen Rest aus der Gruppe von substituiertem Benzyl der Formel XI oder substituiertem Benzoyl der Formel XII darstellt, $R^1$ bis $R^5$ und A die Bedeutung gemäß Anspruch 1 haben.

Zu weiteren bevorzugten Verbindungen zählen:

Dimethyl-{[2-(2-methylbenzyloxy)cyclohexyl]phenylmethyl}amin und das entsprechende Hydrochlorid

[2-(Dimethylaminophenylmethyl)cyclohexyl]-4-trifluormethylbenzoat und das entsprechende Hydrochlorid

[2-(Dimethylaminophenylmethyl)cyclohexyl]-4-methoxybenzoat und das entsprechende Hydrochlorid

{[2-(2-Chlorbenzyloxy)cyclohexyl]-(2-chlorphenyl)-methyl}dimethylamin und das entsprechende Hydrochlorid

{(2-Chlorphenyl)-[2-(4-methylbenzyloxy)cyclohexyl]-methyl}dimethylamin und das entsprechende Hydrochlorid

{[2-(4-Fluorbenzyloxy)cyclohexyl]phenylmethyl}dimethylamin und das entsprechende Hydrochlorid

In einer besonderen Ausführungsform der Erfindung werden die Enantiomere einer erfindungsgemäßen Verbindung in einem nicht-äquimolaren Verhältnis, wobei der Anteil eines Enantiomers am Enantiomerengemisch vorzugsweise 5 bis 45 Massenprozent beträgt, als Wirkstoff in einem Arzneimittel eingesetzt, welches gegebenenfalls weitere Wirkstoffe enthalten kann.

Der Ausdruck "$C_{1-6}$-Alkyl" bedeutet im Rahmen der vorliegenden Erfindung geradkettige oder verzweigte Kohlenwasserstoffe mit 1 bis 6 Kohlenstoffatomen. Beispielhaft seien Methyl, Ethyl, Propyl, Isopropyl, n-Butyl, sek-Butyl, tert-Butyl, n-Pentyl, Neopentyl und n-Hexyl genannt.

Der Ausdruck "$C_{3-7}$-Cycloalkyl" bedeutet im Rahmen der vorliegenden Erfindung gesättigte cyclische Kohlenwasserstoffe oder geradkettige oder verzweigte Alkylreste, die gesättigte cyclische Kohlenwasserstoffe enthalten, mit insgesamt 3 bis 7 Kohlenstoffatomen. Beispielhaft seien Cyclopropyl, Cyclopropylmethyl, Methylcyclopropyl, Cyclobutyl, 1-Cyclopropylethyl, 2-Cyclopropylethyl, Cyclopentyl, Cyclopentylmethyl, Cyclohexyl oder Cycloheptyl erwähnt.

Ein weiterer Gegenstand der vorliegenden Erfindung sind Verfahren zur Herstellung von Verbindungen der allgemeinen Formel **I**. Zur Herstellung der Verbindungen der Formel I setzt man die Mannichbasen der Formel **II** mit einem geeigneten Nukleophil, wie beispielsweise einer metallorganischen Verbindungen ($H_3C$)Y in der Y beispielsweise MgCl, MgBr, MgI oder Li bedeutet, oder einem Reduktionsmittel wie beispielsweise Natriumborhydrid, Natriumcyanoborhydrid, Lithiumaluminiumhydrid, Diisobutylaluminiumhydrid oder einem komplexen Analogon dieser Verbindungen bei Temperaturen zwischen -70 °C und +110 °C umsetzt. Ether oder Ester der allgemeinen Formel **I** lassen sich aus den entsprechenden Alkohole nach Standardmethoden durch Umsetzung der Alkohole mit entsprechenden Benzyloder Benzoylhalogeniden in Gegenwart anorganischer oder organischer Basen erhalten. Ester können auch durch Kondensation der Alkohole mit Carbonsäuren erhalten werden (R.C. Larock; *Comprehensive Organic Transformations*; VCH Publishers; New York, Weinheim, Cambridge 1989).

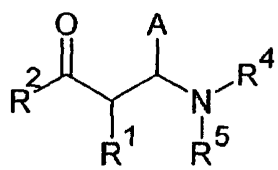

II

Die Umsetzung einer Mannichbase der Formel **II** mit einer Grignardverbindung MeY, in der Y MgCl, MgBr oder MgI bedeutet, oder mit einer lithiumorganischen Verbindung MeLi kann in einem aliphatischen Ether, beispielsweise Diethylether und/oder Tetrahydrofuran, einem Kohlenwasserstoff, beispielsweise Hexan oder Toluol, oder Gemischen aus Kohlenwasserstoffen und aliphatischen Ethern, bei Temperaturen zwischen -70 °C und +110 °C durchgeführt werden. Lithiumorganische Verbindungen MeLi lassen sich aus Organohalogenverbindungen MeZ, in der Z Cl, Br oder I bedeutet, durch Umsetzung mit beispielsweise einer n-Butyllithium/Hexan-Lösung durch Halogen-Lithiumaustausch erhalten.

Bei der Umsetzung einer Mannichbase der Formel II mit einer metallorganischen Verbindung MeY werden in Abhängigkeit von den Reaktionsbedingungen bevorzugt tertiäre Alkohol mit der relativen Konfiguration der Formel **Ia** erhalten, in denen die Aminoarylmethylgruppe *cis* zur Hydroxylgruppe angeordnet ist, wenn $R^1$ und $R^2$ ein Ringsystem bilden. Bei offenkettigen Systemen wird die analoge relative Stereochemie erhalten, die als *anti* zu spezifizieren ist. Die Verbindungen der Formel **Ia** lassen sich durch säulenchromatographische Trennung oder durch Kristallisation, auch ihrer Salze, beispielsweise der Hydrochloride, diastereomerenrein erhalten.

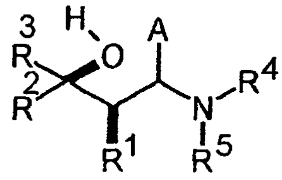

Ia

Die Umsetzung einer Mannichbase der Formel **II** mit einem Reduktionsmittel kann in Alkoholen, Wasser, einem Ether, einem Kohlenwasserstoff, einem Halogenkohlenwasserstoff oder Gemischen dieser Lösungsmittel bei Temperaturen zwischen -70 °C und + 110 °C durchgeführt werden. Die Reaktionsbedingungen können dabei so gewählt werden, daß eines der beiden möglichen Stereoisomere **Ib** und **Ic** bevorzugt bzw. ausschließlich erhalten wird.

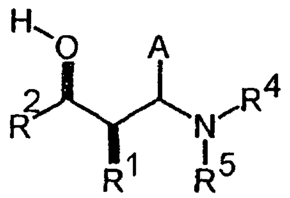

Ib

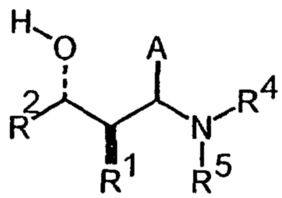

Ic

Die Mannichbasen der Formel **II** lassen sich durch Umsetzung von Enaminen der Formel **III** mit einem Imminiumsalz der Formel **IV,** in dem Y beispielsweise $Cl^-$, $AlCl_4^-$, $Br^-$ oder $I^-$ bedeutet, erhalten.

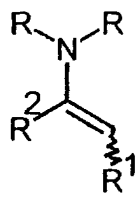

III

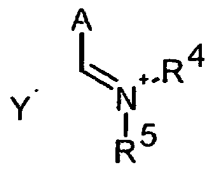

IV

Die Enamine werden nach literaturbekannten Verfahren aus Ketonen der Formel **V** und sekundären Aminen, beispielsweise Dimethylamin, Pyrrolidin, Piperidin oder Morpholin, hergestellt (Acta Chem. Scand. B 38 (1984) 49-53). Die Imminiumsalze werden nach literaturbekannten Verfahren durch Umsetzung von Aminalen der Formel VI mir Säurechloriden, beispielsweise Acetylchlorid oder Thionylchlorid, hergestellt (Houben-Weyl - Methoden der Organischen Chemie, E21b (1995) 1925-1929).

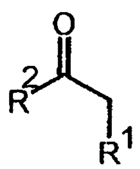

V

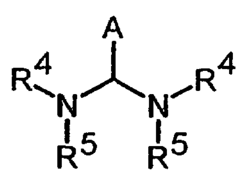

VI

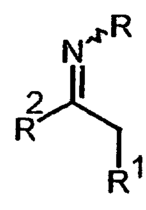

VII

Die Imminiumsalze der Formel **IV** müssen dabei nicht isoliert werden, sondern können in situ erzeugt und mit Enaminen der Formel **III** zu Mannichbasen der Formel **II** umgesetzt werden (Angew. Chem. 106 (1994) 2531-2533). Aufgrund der der Keto-Enol-Tautomerie analogen Enamin-Imin-Tautomerie sind statt der Enamine der Formel **III** auch Imine der Formel **VII** einsetzbar. Alternativ können Ketone der Formel **V** auch direkt mit Imminiumsalzen der Formel **IV** umgesetzt werden.

Mannichbasen der Formel **II** können aber auch durch Umsetzung von Enaminen der Formel **III** mit einem aromatischen Aldehyd der Formel **VIII** und einem sekundären Amin $HNR^4R^5$, auch in Form des korrespondierenden Hydrochlorids $HNR^4R^5$, HCl, in Gegenwart von Triethylamin, Chlortrimethylsilan und Natriumiodid direkt hergestellt werden (Synlett (1997) 974-976).

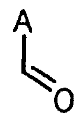

VIII

Die Mannichbasen der Formel **II** werden mit den oben beschriebenen Verfahren in Abhängigkeit von den Reaktionsbedingungen bevorzugt mit der relativen Konfiguration der Formel IIa erhalten, in denen die Aminogruppe *anti* zu $R^1$ angeordnet ist. Die Verbindungen der Formel **IIa** lassen sich durch Kristallisation, auch ihrer Salze, beispielsweise der Hydrochloride, oder durch chromatographische Trennung diastereomerenrein erhalten.

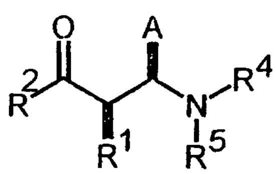

IIa

Weniger stereoselektiv verläuft dagegen die Darstellung von Mannichbasen der Formel **II** durch 1,4-Addition sekundärer Amine $HNR^4R^5$ an Enone der Formel **IX**, die aus der Aldolkondensation von Ketonen der Formel **V** mit aromatischen Aldehyden der Formel **VIII** erhalten werden (US-Patent 4,017,637). Diese Vorgehensweise eignet sich daher zur Darstellung der anderen möglichen Stereoisomeren.

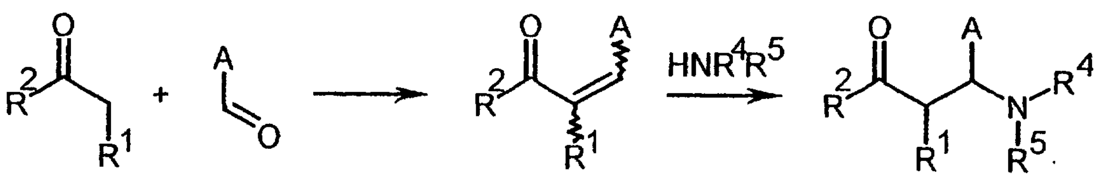


V VIII IX II

Die Bedeutung der Reste $R^1$ bis $R^5$ bzw. A entspricht der Bedeutung gemäß Formel I.

Werden chirale Amine zur Darstellung von Enaminen der Formel **III** oder Iminen der Formel **VII** eingesetzt, so können in der nachfolgenden Mannichreaktion enantiomerenangereicherte bis enantiomerenreine Mannichbasen der Formel **II** erhalten werden (Houben-Weyl - Methoden der Organischen Chemie, E21b (1995) 1925-1929).

3-Amino-3-arylpropan-1-ol-Derivate der allgemeinen Formel **I**, die mit einem Phenol substituiert sind, lassen sich beispielsweise aus den entsprechenden Methylether-Derivaten mit Diisobutylaluminiumhydrid in einem aromatischen Kohlenwasserstoff, beispielsweise Toluol, bei einer Temperatur zwischen 60 und 130 °C herstellen (Synthesis (1975) 617-630).

Die Verbindungen der Formel I lassen sich mit physiologisch verträglichen Säuren, beispielsweise Salzsäure,

Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in wäßriger Lösung.

Die der Formel I entsprechenden Substanzen sind toxikologisch unbedenklich, so daß sie sich als pharmazeutischer Wirkstoff in Arzneimittel eignen. Ein weiterer Gegenstand der vorliegenden Erfindung sind daher Arzneimittel enthaltend wenigstens eine Verbindung der allgemeinen Formel I als Wirkstoff. Vorzugsweise eignen sich die erfindungsgemäßen Arzneimittel als Analgetika.

Biochemische Untersuchungen haben ergeben, daß die erfindungsgemäßen Substanzen neben ihrer analgetischen Wirkung auch ausgeprägte Affinität zur Bindungsstelle 2 des Natriumkanals (BTX-Bindung) und zur Benzothiazepin-Bindungsstelle des L-Typ Kalziumkanals (Diltiazem-Bindung) zeigen. Die erfindungsgemäßen Substanzen eignen sich daher neben der besonders bevorzugten Verwendung in der Schmerztherapie auch zur Verwendung als Lokalanästetikum und/oder Antiarrhythmikum und/oder Antiemetikum und/oder Nootropikum (Neurotropikum) und/oder zur Behandlung/Therapie von cardiovaskulären Erkrankungen und/oder Harninkontinenz und/oder Diarrhöe und/oder Pruritus und/oder Entzündungen.

Die pharmazeutischen Formulierungen enthalten neben mindestens einem 3-Amino-3-arylpropan-1-ol-Derivat der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der Formel I in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der Formel I verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,5 bis 500 mg/kg wenigstens eines 3-Amino-3-arylpropan-1-ol-Derivats der Formel I appliziert.

**Pharmakologische Untersuchungen**

**Analgesieprüfung im Writhing-Test an der Maus**

Die Untersuchung auf analgetische Wirksamkeit wurde im Phenylchinon-induzierten Writhing an der Maus (modifiziert nach I.C. Hendershot und J. Forsaith (1959) J. Pharmacol. Exp. Ther. 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der Schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Alle Substanzen wurden in der Standarddosierung von 10 mg/kg getestet. Die prozentuale Hemmung (%Hemmung) der Writhingreaktion durch eine Substanz wurde nach folgender Formel berechnet:

$$\%\ \text{Hemmung} = 100 - \frac{\text{Writhingreaktionen der behandelten Tiere}}{\text{Writhingreaktionen der Kontrolltiere}} * 100$$

Für einige Substanzen wurde aus der dosisabhängigen Abnahme der Writhingreaktionen im Vergleich zu parallel untersuchten Phenylchinon-Kontrollgruppen mittels Regressionsanalyse (Auswerteprogramm Martens EDV Service, Eckental) die $ED_{50}$-Werte mit 95 % Vertrauensbereich der Writhingreaktion berechnet.

Alle untersuchten erfindungsgemäßen Verbindungen zeigten eine ausgeprägte analgetische Wirkung. Die Ergebnisse sind in Tabelle 1 zusammengefaßt.

Tabelle 1:

| Analgesieprüfung im Writhing-Test an der Maus | |
|---|---|
| Beispiel | %Hemmung der Writhingreaktion bei 10 mg/kg intravenös |
| 1 | 85 |
| 2 | 48 |
| 3 | 43 |
| 4 | 32 |
| 5 | 74 |
| 6 | 68 |

**Biochemische Untersuchungen**

**Bindungsuntersuchungen am L-Kalziumkanal: Benzothiazepin-Bindungsstelle (Diltiazem-Bindung)**

Das biologische Membranmaterial wurde aus dem Cerebrocortex der Ratte isoliert. Als Ligand wurde [$^3$H]-cis-(+)-Diltiazem (5 nM im Ansatz) verwendet. Inkubation für 20 Minuten bei 25 °C. Als unspezifische Bindung ist die Radioaktivität definiert, die bei Anwesenheit von (±)-Diltiazem ($10^{-6}$ M im Ansatz) gemessen wird. Der nicht gebundene Anteil des radioaktiven Liganden wird nach Abschluß der Inkubation mit Hilfe eines Filtrationsprozesses über Whatman Glasfiber GF/B-Membranen abgetrennt. Die Membranen werden anschließend nach einem Waschprozeß am β-Counter vermessen. Die Methode ist in Anlehnung an die Veröffentlichung von Schoemaker und Langer (H. Schoemaker und S.Z. Langer (1985) Eur. J. Pharmacol. 111, 273-277) erstellt worden. Der $K_D$-Wert für diese hoch affine Bindungsstelle wurde mit 4,10 ± 0,75 nM bestimmt (N = 3, d.h. Mittelwerte ± SEM aus 3 unabhängigen Versuchsreihen, die in Dreifach-Parallelversuchen durchgeführt worden sind).

**Bindungsuntersuchungen am Natriumkanal: Bindungsstelle 2 (BTX-Bindung)**

Die Bindungsstelle 2 des Natriumkanals ist die sogenannte Batrachotoxin-(BTX) Bindungsstelle. Als Ligand wurde [$^3$H]-Batrachotoxinin A20 α-Benzoat (10 nM im Ansatz) eingesetzt. Diese Ionenkanal-Partikel (Synaptosomen) wurden aus dem Ratten Cerebrocortex nach Gray und Whittaker (E.G. Gray und V.P. Whittaker (1962) J. Anat. 76, 79-88) angereichert. Als unspezifische Bindung ist die Radioaktivität definiert, die in Gegenwart von Veratridin (0,3 mM im Ansatz) gemessen wird. Inkubation bei 37°C für 120 min. Die Assaybedingungen sind nach der Veröffentlichung von Pauwels, Leysen und Laduron (P.J. Pauwels, J.E. Leysen und P.M. Laduron (1986) Eur. J. Pharmacol. 124, 291-298) durchgeführt worden.

Der $K_D$-Wert für diese Bindungsstelle liegt bei 24,63 ± 1,56 nM. (N = 3, d.h. Mittelwerte ± SEM aus 3 unabhängigen Versuchsreihen, die in Dreifach-Parallelversuchen durchgeführt worden sind).

**Auswertung**

Neben der prozentualen Hemmung der Testsysteme bei fixen Testsubstanzkonzentrationen (10 μM im Ansatz), wurden Dosisabhängigkeiten überprüft. Hierbei werden $IC_{50}$-Werte erhalten, die gemäß der "Cheng-Prusoff Gleichung" (Y.C. Cheng und W.H. Prusoff (1973) Biochem. Pharmacol. 22, 3099-3108) in Inhibitorkonstanten ($K_i$) umgerechnet werden können. Die $IC_{50}$ Werte wurden mit Hilfe des Computer-Programms "Figure P" (Version 6.0, Biosoft, Cambridge, England) erhalten. Km-Werte wurden gemäß Lineweaver und Burk (H. Lineweaver und D. Burk (1934) J. Am. Chem. Soc. 56, 658-666) berechnet. Um $K_D$-Werte darzustellen, ist das Computer-Programm "Ligand" (Version 4, Biosoft, England) angewendet worden.

Die Ergebnisse der biochemischen Untersuchungen sind in Tabelle 2 zusammengefaßt.

Tabelle 2

| Biochemie | | |
|---|---|---|
| Beispiel | Diltiazem-Bindung bei 10 μM | BTX-Bindung bei 10 μM |
| **1** | 82 | 99 |
| **2** | | 98 |
| **3** | 65 | 89 |
| **4** | 73 | 92 |
| **5** | 89 | 98 |
| **6** | 86 | 97 |

**Beispiele**

Die folgenden Beispiele dienen zur näheren Erläuterung des erfindungsgemäßen Verfahrens.

Die Ausbeuten der hergestellten Verbindungen sind nicht optimiert.

Alle Temperaturen sind unkorrigiert.

Die Angabe Ether bedeutet Diethylether.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Racemattrennungen wurden auf einer Chiracel OD Säule 250 x 4,6 mm mit Vorsäule der Firma Daicel durchgeführt.

Die Mischungsverhältnisse der Laufmittel für alle chromatographischen Untersuchungen sind stets in Volumen/Volumen angegeben.

RT bedeutet Raumtemperatur, Vol.% Volumenprozent, m% Massenprozent und %ee Enantiomerenüberschuß in Prozent.

**Beispiel 1**

Dimethyl-{[2-(2-methyl-benzyloxy)cyclohexyl]phenylmethyl}amin Hydrochlorid

1. Stufe Benzylidendimethylammoniumchlorid

10 g (56 mmol) N,N,N',N'-Tetramethyl-C-phenylmethandiamin (J. Am. Chem. Soc. 77 (1955) 1114-1116) wurden in 100 ml Ether gelöst und im Eisbad auf 0°C gekühlt. Es wurden unter Stickstoff 4,0 ml (56 mmol) Acetylchlorid zugetropft. Nach den ersten Tropfen fiel ein weißes Salz aus, die Temperatur erhöhte sich leicht. Nach 15 Stunden bei RT wurde abdekantiert, der Feststoff dreimal mit je 100 ml Ether gewaschen, über eine Schutzgasfritte unter Stickstoff filtriert und im Ölpumpenvakuum bis zur Gewichtskonstanz getrocknet. Auf diese Weise wurden 7,7 g Benzylidendimethylammoniumchlorid (80,9 % der Theorie) erhalten.

2. Stufe 2-(Dimethylaminophenylmethyl)cyclohexanon

7,1 ml (44 mmol) 1-(Pyrrolidino)-1-cyclohexen wurden in 45 ml Dichlormethan gelöst und unter Stickstoff mit einem Trockeneis/Isopropanol-Bad auf -70 °C gekühlt. Unter Rühren wurden 7,5 g (44 mmol) Benzylidendimethylammoniumchlorid aus Stufe 1 zugegeben, die Mischung innerhalb von zwei bis drei Stunden auf -30°C erwärmt und 15 Stunden bei dieser Temperatur gelagert. Zur Aufarbeitung wurden 60 ml halbkonzentrierte Salzsäure zugegeben und 5 Minuten nachgerührt. Bei RT wurde mit 50 ml Ether gewaschen, die wässrige Phase mit 440 ml Ammoniaklösung (25 Vol.%) versetzt und schnell dreimal mit je 150 ml Ether extrahiert. Die vereinigten organischen Extrakte wurden über Natriumsulfat getrocknet, filtriert und am Rotationsverdampfer ohne Wärmezufuhr (500 bis 10 mbar) eingeengt. Auf diese Weise wurden 10,1 g Rohbase (99,5 % der Theorie) erhalten. 9,81 g (42,4 mmol) der Rohbase wurden in 83 ml 2-Butanon gelöst und nacheinander 0,76 ml (42,2 mmol) Wasser und 5,36 ml (42,4 mmol) Chlortrimethylsilan zugegeben. Der Ansatz wurde 15 Stunden bei RT aufbewahrt, der ausgefallene Feststoff abgesaugt, mit kleinen Portionen Ether gewaschen und im Ölpumpenvakuum bis zur Gewichtskonstanz getrocknet. Auf diese Weise wurden 8,92 g des Hydrochlorids von 2-(Dimethylaminophenylmethyl)cyclohexanon (78,6 % der Theorie) erhalten.

3. Stufe 2-(Dimethylaminophenylmethyl)cyclohexanol

Zu 26 ml (39 mmol) Diisobutylaluminiumhydrid (1,5 M in Toluol) tropfte man unter Stickstoff bei RT 3,0 g (13,0 mmol) des nach Beispiel 1 (2. Stufe) hergestellten 2-(Dimethylaminophenylmethyl)cyclohexanons, gelöst in 26 ml Toluol. Es wurde unter Rühren 15 Stunden zum Rückfluß erhitzt. Zur Aufarbeitung wurden 13 ml Ethanol und 13 ml Wasser langsam zugetropft, die Suspension mehrere Stunden bei 0°C gelagert, durch eine Glasfritte filtriert und mehrmals mit wenig Toluol gewaschen. Das Filtrat wurde am Rotationsverdampfer (500 bis 10 mbar) eingeengt, Lösungsmittelreste im Hochvakuum entfernt (ca. 0,1 mbar). Es wurden 2,62 g (86,6% der Theorie) 2-(Dimethylaminophenylmethyl)cyclohexanol erhalten.

4. Stufe Dimethyl-{[2-(2-methylbenzyloxy)cyclohexyl]phenylmethyl}amin Hydrochlorid

1,00 g (4,29 mmol) 2-(Dimethylaminophenylmethyl)cyclohexanol wurden in 5,0 ml Dimethylsulfoxid p.a. gelöst und unter Stickstoff 577 mg (5,14 mmol) Kalium-*tert*.-butylat zugegeben, gelöst in 1,0 ml Dimethylsulfoxid p.a.. Die Reaktionsmischung wurde für 30 Minuten auf 50°C erhitzt, 904 mg (6,43 mmol) 2-Methylbenzylchlorid zugetropft und weitere 15 Stunden bei 50°C gerührt. Zur Aufarbeitung wurden 10 ml Wasser zugegeben und dreimal mit je 15 ml Ether extrahiert. Die vereinigten Extrakte wurden mit je 10 ml Kaliumhydroxidlösung (2 M) und Wasser gewaschen und dreimal mit je 25 ml Salzsäure (5 m%) extrahiert. Die vereinigten sauren Extrakte wurden mit Natronlauge (32 m%) alkalisch gestellt (pH $\geq$ 10) und dreimal mit je 25 ml Dichlormethan extrahiert. Die vereinigten organischen Extrakte wurden über Natriumsulfat getrocknet, filtriert und am Rotationsverdampfer (500 bis 10 mbar) eingeengt. Es wurden 414 mg Rohbase erhalten. Die Rohbase wurde in 4 ml 2-Butanon gelöst und nacheinander 11 µl (0,61 mmol) Wasser und 155 µl (1,22 mmol) Chlortrimethylsilan zugegeben. Der Ansatz wurde 15 Stunden bei RT aufbewahrt, der ausgefallene Feststoff abgesaugt, mit kleinen Portionen Ether gewaschen und im Ölpumpenvakuum bis zur Gewichtskonstanz getrocknet. Auf diese Weise wurden 221 mg des Hydrochlorids von Dimethyl-{[2-(2-methylbenzyloxy)-cyclohexyl] phenylmethyl}amin (13,8 % der Theorie) erhalten, das sich beim Erhitzen ab 79°C zersetzt.

**Beispiel 2**

[2-(Dimethylaminophenylmethyl)cyclohexyl]-4-trifluormethylbenzoat Hydrochlorid

1. Stufe 2-(Dimethylaminophenylmethyl)cyclohexanol

Zu 80,0 g (299 mmol) des nach Beispiel 1 (2. Stufe) hergestellten 2-(Dimethylaminophenylmethyl)cyclohexanons wurden in 650 ml Methanol gelöst, portionsweise insgesamt 28,3 g (747 mmol) Natriumborhydrid zugegeben und eine Stunde nachgerührt. Zur Aufarbeitung wurden 680 ml verdünnte Salzsäure (1 N) zugegeben und mit 500 ml Ether extrahiert. Die wäßrige Phase wurde mit Ammoniaklösung (25 Vol.%) alkalisch gestellt (pH $\geq$ 10) und dreimal mit je 250 ml Ether extrahiert. Die vereinigten organischen Extrakte wurden über Natriumsulfat getrocknet, filtriert und am Rotationsverdampfer (500 bis 10 mbar) eingeengt. Es wurden 65,2 g (94 % der Theorie) 2-(Dimethylamimophenylmethyl)-cyclohexanol erhalten.

2. Stufe [2-(Dimethylaminophenylmethyl)cyclohexyl]-4-trifluormethylbenzoat Hydrochlorid

1,34 g (6,43 mmol) 4-(Trifluormethyl)benzoylchlorid wurden in 4 ml Dichlormethan gelöst und bei - 10°C (Methanol/Eis-Kühlbad) 870 mg (8,57 mmol) Triethylamin hinzugegeben. Anschließend wurden 1,0 g (4,29 mmol) 2-(Dimethylaminophenylmethyl)cyclohexanol, gelöst in 2 ml Dichlormethan, zugetropft und 15 Stunden nachgerührt. Zur Aufarbeitung wurden 2 ml Kaliumhydroxidlösung (0,5 N) zugegeben, die organische Phase abgetrennt, über Natriumsulfat getrocknet, filtriert und am Rotationsverdampfer (500 bis 10 mbar) eingeengt. Es wurden 1,7 g Rohbase erhalten. Aus der Rohbase wurde nach Beispiel 1 (4. Stufe) mit Chlortrimethylsilan/Wasser in 2-Butanon 877 mg des Hydrochlorids von [2-(Dimethylaminophenylmethyl)cyclohexyl]-4-trifluormethylbenzoat (34% der Theorie) mit einem Schmelzpunkt über 230°C erhalten.

**Beispiel 3**

[2-(Dimethylaminophenylmethyl)cyclohexyl]-4-methoxybenzoat Hydrochlorid

1,06 g (6,43 mmol) 4-Methoxybenzoylchlorid wurden in 4 ml Dichlormethan gelöst und bei - 10°C (Methanol/Eis-Kühlbad) 870 mg (8,57 mmol) Triethylamin hinzugegeben. Anschließend wurden 1,0 g (4,29 mmol) des nach Beispiel 2 (1. Stufe) hergestellten 2-(Dimethylaminophenylmethyl)cyclohexanols, gelöst in 2 ml Dichlormethan, zugetropft und 15 Stunden nachgerührt. Zur Aufarbeitung wurden 2 ml Kaliumhydroxidlösung (0,5 N) zugegeben, die organische Phase abgetrennt, über Natriumsulfat getrocknet, filtriert und am Rotationsverdampfer (500 bis 10 mbar) eingeengt. Es wurden 1,78 g Rohbase erhalten. Aus der Rohbase wurde nach Beispiel 1 (4. Stufe) mit Chlortrimethylsilan/Wasser in 2-Butanon 1,00 g des Hydrochlorids von [2-(Dimethylaminophenylmethyl)cyclohexyl]-4-methoxybenzoat (58% der Theorie) mit einem Schmelzpunkt über 230°C erhalten.

**Beispiel 4**

{[2-(2-Chlorbenzyloxy)cyclohexyl]-(2-chlorphenyl)methyl}dimethylamin Hydrochlorid

1. Stufe 2-[(2-Chlorphenyl)dimethylaminomethyl]cyclohexanon

Zu 471 ml (469 mmol) Natriumiodidlösung (1 M in Acetonitril), mit einem Eisbad auf 0°C gekühlt, wurden unter Rühren 17,4 g (213 mmol) frisch getrocknetes Dimethylamin-Hydrochlorid gegeben, 60 ml (427 mmol) Triethylamin und 60 ml (469 mmol) Chlortrimethylsilan zugetropft und eine Stunde bei RT nachgerührt. Bei Eiskühlung wurden 24 ml (213 mmol) 2-Chlorbenzaldehyd zugegeben und noch eine Stunde bei RT weitergerührt. Es wurde erneut mit einem Eisbad auf 0°C gekühlt, 34 ml (213 mmol) 1-(Pyrrolidino)-1-cyclohexen zugegeben und zwei Stunden bei RT weitergerührt. Zur Aufarbeitung wurde der Ansatz bei Eiskühlung mit 300 ml halbkonzentrierter Salzsäure versetzt, 10 Minuten gerührt, zweimal mit je 300 ml Ether gewaschen und mit 770 ml verdünnter Ammoniaklösung (5 Vol.%) alkalisch gestellt (pH ca. 9). Es wurde dreimal mit je 300 ml Ether extrahiert, die vereinigten organischen Extrakte über Natriumsulfat getrocknet, filtriert und am Rotationsverdampfer (500 bis 10 mbar) ohne Wärmezufuhr eingeengt. Auf diese Weise wurden 38,3 g 2-[(2-Chlorphenyl)dimetlylaminemethyl]cyclohexanon (68% der Theorie) erhalten.

2. Stufe 2-[(2-Chlorphenyl)dimethylaminomethyl]cyclohexanol Hydrochlorid

10,0 g (37,6 mmol) 2-[(2-Chlorphenyl)dimethylaminomethyl]-cyclohexanon wurden in 190 ml Methanol gelöst und 2,85 g (75,2 mmol) Natriumborhydrid portionsweise eingetragen. Zur Aufarbeitung wurden unter Rühren 170 ml Salzsäure (1 M) zugesetzt und mit 100 ml Ether extrahiert. Mit 15 ml Ammoniaklösung (25 Vol.%) wurde alkalisch gestellt (pH ≥ 10) und dreimal mit je 100 ml Ether extrahiert. Die vereinigten Extrakte wurden über wasserfreiem Natriumsulfat getrocknet, filtriert und am Rotationsverdampfer (500 bis 10 mbar) eingeengt. Es wurden 8,10 g Rohbase (80,3% der Theorie) erhalten. Aus 1,98 g (7,39 mmol) dieser Base wurden nach Beispiel 1 (4. Stufe) mit Chlortrimethylsilan/Wasser in 2-Butanon 1,74 g des Hydrochlorids von 2-[(2-Chlorphenyl)dimethylaminomethyl]cyclohexanol (78% der Theorie) erhalten, das sich ab 131°C zersetzt.

3. Stufe {[2-(2-Chlorbenzyloxy)cyclohexyl]-(2-chlorphenyl)methyl}dimethylamin Hydrochlorid

902 mg (5,60 mmol) 2-Chlorbenzylchlorid und 1,0 g (3,73 mmol) 2-[(2-Chlorphenyl)dimethylaminomethyl] cyclohexanol wurden in 6,0 ml Dimethylsulfoxid p.a. gelöst, unter Stickstoff 503 mg (4,48 mmol) festes Kalium-*tert*. butylat zugegeben und für 15 Stunden auf 100°C erhitzt. Zur Aufarbeitung wurden 10 ml Wasser zugegeben und dreimal mit je 15 ml Ether extrahiert. Die vereinigten Extrakte wurden mit je 10 ml Kaliumhydroxidlösung (2 M) und Wasser gewaschen und dreimal mit je 25 ml Salzsäure (5 m%) extrahiert. Die vereinigten sauren Extrakte wurden mit Natronlauge (32 m%) alkalisch gestellt (pH ≥ 11) und dreimal mit je 25 ml Dichlormethan extrahiert. Die vereinigten organischen Extrakte wurden über Natriumsulfat getrocknet, filtriert und am Rotationsverdampfer (500 bis 10 mbar) eingeengt. Es wurden 277 mg Rohbase erhalten. Aus dieser Rohbase wurden nach Beispiel 1 (4. Stufe) mit Chlortrimethylsilan/Wasser in 2-Butanon 151 mg {[2-(2-Chlorbenzyloxy)cyclohexyl]-(2-chlorphenyl)methyl}dimethylamin Hydrochlorid (9,4% der Theorie) mit einem Schmelzbereich von 108 - 100°C erhalten.

**Beispiel 5**

{[2-(3-Fluorbenzyloxy)cyclohexyl]phenylmethyl}dimethylamin Hydrochlorid

1,00 g (4,29 mmol) des nach Beispiel 1 (3. Stufe) hergestellten 2-(Dimethylaminophenylmethyl)cyclohexanols wurden in 5 ml Dimethylsulfoxid p.a. gelöst und bei 50°C 503 mg (4,48 mmol) Kalium-*tert*.-butylat zugegeben. Dann wurde auf 100°C erhitzt und 323 µl (2,61 mmol) 3-Fluorbenzylchlorid zugegeben. Diese Zugabe wurde noch zweimal nach jeweils zwei Stunden wiederholt und die Reaktionsmischung anschließend für weitere 15 Stunden auf 100°C erhitzt. Die Aufarbeitung wurde nach Beispiel 1 (4. Stufe) durchgeführt, wodurch 166 mg {[2-(3-Fluorbenzyloxy)cyclohexyl]phenylmethyl}dimethylamin Hydrochlorid erhalten wurden (11% der Theorie), das sich beim Erhitzen ab 203°C zersetzt.

**Beispiel 6**

{[2-(4-Fluorbenzyloxy)cyclohexyl]phenylmethyl}dimethylamin Hydrochlorid

Zu einer Suspension von 108 mg (4,48 mmol) Natriumhydrid in 1 ml Dimethylformamid p.a. wurde eine Lösung von 1,00 g (4,29 mmol) des nach Beispiel 2 (1. Stufe) hergestellten 2-(Dimethylaminophenylmethyl)cyclohexanols in 5 ml Dimethylformamid p.a. gegeben. Die Reaktionsmischung wurde auf 100°C erhitzt und 323 µl (2,61 mmol) 3-Fluorbenzylchlorid zugegeben. Diese Zugabe wurde noch zweimal nach jeweils zwei Stunden wiederholt und die Reaktionsmischung anschließend für weitere 15 Stunden auf 100°C erhitzt. Die Aufarbeitung wurde nach Beispiel 1 (4. Stufe) durchgeführt, wodurch 393 mg ([2-(4-Fluorbenzyloxy)cyclohexyl]-phenylmethyl}dimethylamin Hydrochlorid erhalten wurden (24% der Theorie), das sich beim Erhitzen ab 210°C zersetzt.

## Patentansprüche

**1.** 3-Amino-3-arylpropan-1-ol-Derivate der allgemeinen Formel I

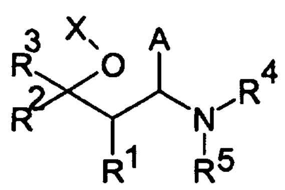

I,

worin

$R^1$, $R^2$ ,jeweils unabhängig voneinander, $C_{1-6}$-Alkyl oder $R^1$ und $R^2$ zusammen einen $(CH_2)_{2-6}$-Ring bilden, der auch benzokondensiert oder phenylsubstituiert sein kann,

$R^3$ H oder Methyl,

$R^4$, $R^5$ ,jeweils unabhängig voneinander, $C_{1-6}$-Alkyl, $C_3$-$C_6$-Cycloalkyl, Phenyl, Benzyl, Phenethyl oder $R^4$ und $R^5$ zusammen einen $(CH_2)_{3-6}$- oder $CH_2CH_2OCH_2CH_2$-Ring bilden,

A einen Rest aus der Gruppe von substituiertem Phenyl der Formel XIII

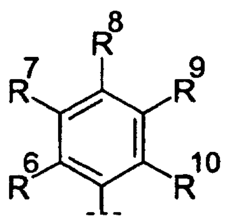

XIII,

oder einen einfach oder mehrfach mit Resten, die ausgewählt sind aus den für $R^6$ bis $R^{10}$ angegebenen Bedeutungen, substituierten Furanyl-, Thiophenyl-, Pyrrolyl-, Pyridinyl-, Pyrimidinyl-, Chinolinyl-, Isochinolinyl-, Phthalazinyl- oder Chinazolinyl-Rest darstellt,
worin

$R^6$ bis $R^{10}$ ,jeweils unabhängig voneinander, H, F, Cl, Br, I, $CF_3$, $OR^{11}$, $OCF_3$, $SR^{11}$, $SO_2CH_3$, $SO_2CF_3$, $C_{1-6}$-Alkyl, Phenyl, CN, $COOR^{11}$, $NO_2$ oder $R^6$ und $R^7$ oder $R^7$ und $R^8$ zusammen einen $OCH_2O$- oder $OCH_2CH_2O$-Ring bilden,

$R^{11}$ $C_{1-6}$-Alkyl, Phenyl, Benzyl, Phenethyl

bedeutet,

X ein substituiertes Benzyl der Formel XI

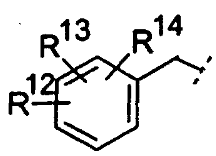

XI

oder ein substituiertes Benzoyl der Formel XII

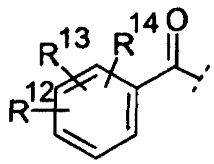

XII,

worin

$R^{12}$ bis $R^{14}$ jeweils unabhängig voneinander, H, F, Cl, Br, $CHF_2$, $CF_3$, $OR^{11}$, $SR^{11}$, $OCF_3$, $SO_2CH_3$, $SO_2CF_3$, $C_{1-6}$-Alkyl, Phenyl, CN, $COOR^{11}$, $NO_2$ mit

$R^{11}$ H, $C_{1-6}$-Alkyl, Phenyl, Benzyl, Phenethyl

bedeuten
und deren Diastereomere oder Enantiomere in Form ihrer Basen oder Salze physiologisch verträglicher Säuren.

**2.** Verbindungen gemäß Anspruch 1, **dadurch gekennzeichnet, daß** $R^1$ und $R^2$ zusammen einen $(CH_2)_{2-6}$-Ring bilden, der benzokondensiert oder phenylsubstituiert sein kann, $R^3$ bis $R^5$, A und X die Bedeutung gemäß Anspruch 1 besitzen.

**3.** Verbindungen gemäß Anspruch 1, **dadurch gekennzeichnet, daß** $R^1$ und $R^2$ zusammen einen $(CH_2)_4$-Ring bilden, der benzokondensiert oder phenylsubstituiert sein kann, $R^3$ bis $R^5$, A und X die Bedeutung gemäß Anspruch 1 besitzen.

**4.** Verbindungen gemäß Anspruch 1, **dadurch gekennzeichnet, daß** $R^3$ Wasserstoff darstellt, $R^1$, $R^2$, $R^4$, $R^5$, A und X die Bedeutung gemäß Anspruch 1 besitzen.

**5.** Verbindungen gemäß Anspruch 1, **dadurch gekennzeichnet, daß** $R^1$ und $R^2$ zusammen einen $(CH_2)_{2-6}$-Ring bilden, der benzokondensiert oder phenylsubstituiert sein kann, $R^3$ Wasserstoff darstellt, $R^4$, $R^5$, A und X die Bedeutung gemäß Anspruch 1 besitzen.

**6.** Verbindungen gemäß Anspruch 1, **dadurch gekennzeichnet, daß** $R^1$ und $R^2$ zusammen einen $(CH_2)_4$-Ring bilden, der benzokondensiert oder phenylsubstituiert sein kann, A einen Rest aus der Gruppe von substituiertem Phenyl der Formel XIII oder Thiophenyl oder Furanyl bedeutet, $R^3$ bis $R^5$ und X die Bedeutung gemäß Anspruch 1 besitzen.

**7.** Verbindungen gemäß Anspruch 1 **dadurch gekennzeichnet, daß** $R^1$ und $R^2$ zusammen einen $(CH_2)_4$-Ring bilden, A einen Rest aus der Gruppe von substituiertem Phenyl der Formel XIII oder Thiophenyl oder Furanyl bedeutet, $R^3$ bis $R^5$ und X die Bedeutung gemäß der Definition des Anspruchs 1 besitzen.

**8.** Verbindungen gemäß Anspruch 1 **dadurch gekennzeichnet, daß** $R^1$ und $R^2$ zusammen einen $(CH_2)_4$-Ring bilden, A Thiophenyl bedeutet, $R^3$ bis $R^5$ und X die Bedeutung gemäß der Definition des Anspruchs 1 besitzen.

**9.** Verbindungen gemäß Anspruch 1 **dadurch gekennzeichnet, daß** $R^1$ und $R^2$ zusammen einen $(CH_2)_4$-Ring bilden, A Furanyl bedeutet, $R^3$ bis $R^5$ und X die Bedeutung gemäß der Definition des Anspruchs 1 besitzen.

**10.** Verbindungen gemäß Anspruch 1 **dadurch gekennzeichnet, daß** X einen Rest aus der Gruppe von substituiertem Benzyl der Formel XI darstellt, $R^1$ bis $R^5$ und A die Bedeutung gemäß Anspruch 1 besitzen.

**11.** Verbindungen gemäß Anspruch 1:

Dimethyl-{[2-(2-methylbenzyloxy)cyclohexyl]phenylmethyl}amin und das entsprechende Hydrochlorid

[2-Dimethylaminophenylmethyl)cyclohexyl]-4-trifluormethylbenzoat und das entsprechende Hydrochlorid

[2-(Dimethylaminophenylmethyl)cyclohexyl]-4-methoxybenzoat und das entsprechende Hydrochlorid

{[2-(2-Chlorbenzyloxy)cyclohexyl]-(2-chlorphenyl)-methyl}dimethylamin und das entsprechende Hydrochlorid

{[2-(3-Fluorbenzyloxy)cyclohexyl]phenylmethyl}dimethylamin und das entsprechende Hydrochlorid

{[2-(4-Fluorbenzyloxy)cyclohexyl]phenylmethyl}dimethylamin und das entsprechende Hydrochlorid.

**12.** Arzneimittel enthaltend als Wirkstoff wenigstens eine Verbindung gemäß den Ansprüchen 1 bis 11.

**13.** Arzneimittel enthaltend als Wirkstoffe ein Gemisch der Enantiomeren einer Verbindung gemäß den Ansprüchen 1 bis 11, wobei die beiden Enantiomeren nicht in äquimolaren Mengen vorliegen, und gegebenenfalls weitere Wirkstoffe.

**14.** Arzneimittel enthaltend als Wirkstoffe ein Gemisch der Enantiomeren einer Verbindung gemäß den Ansprüchen 1 bis 11, wobei eines der Enantiomere einen relativen Anteil zwischen 5 und 45 Massenprozent am Enantiomerengemisch hat, und gegebenenfalls weitere Wirkstoffe.

**15.** Verfahren zur Herstellung einer Verbindung gemäß den Ansprüchen 1 bis 11 **dadurch gekennzeichnet, daß** eine Mannich-Base der allgemeinen Formel II

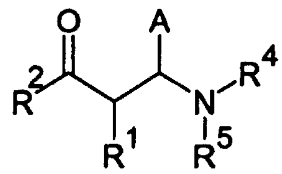

**II,**

worin $R^1$ bis $R^5$ und A die Bedeutung gemäß der allgemeinen Formel I besitzen, mit einer Grignardverbindung der allgemeinen Formel $(H_3C)Y$, worin Y MgCl, MgBr oder MgI bedeutet, oder MeLi oder einem Reduktionsmittel, bevorzugt aus der Gruppe von Natriumborhydrid, Natriumcyanoborhydrid, Lithiumaluminiumhydrid, Diisobutylaluminiumhydrid oder einem komplexen Analogon dieser Verbindungen, zu einem Alkohol der allgemeinen Formeln Id umgesetzt wird

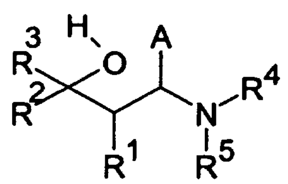

**Id,**

worin
$R^1$ bis $R^5$ und A die Bedeutung wie in der allgemeinen Formel I haben,
ein Alkohol der allgemeinen Formeln Id mit HalX, worin Hal die Bedeutung von Halogenen aus der Gruppe von F, Cl, Br oder I hat und X die Bedeutung gemäß der allgemeinen Formel I hat, in Gegenwart von anorganischen oder organischen Basen in einem Temperaturbereich von 0°-150°C umgesetzt wird oder mit XOH in einem Temperaturbereich von 0°-150°C kondensiert wird und so in eine Verbindung der allgemeinen Formel I überführt wird.

**16.** Verwendung einer Verbindung gemäß den Ansprüchen 1 bis 11 zur Herstellung eines Arzneimittels zur Schmerzbekämpfung.

**17.** Verwendung nach Anspruch 16 zur Herstellung eines Arzneimittels zur Bekämpfung neuropathischer Schmerzen.

**18.** Verwendung nach Anspruch 16 zur Herstellung eines Arzneimittels zur Bekämpfung chronischer Schmerzen.

**19.** Verwendung einer Verbindung gemäß den Ansprüchen 1 bis 11 zur Herstellung eines Arzneimittels mit lokalanästhetischer Wirkung.

**20.** Verwendung einer Verbindung gemäß den Ansprüchen 1 bis 11 zur Herstellung eines Arzneimittels mit antiarrhythmischer Wirkung.

**21.** Verwendung einer Verbindung gemäß den Ansprüchen 1 bis 11 zur Herstellung eines Arzneimittels mit antiemetischer Wirkung.

**22.** Verwendung einer Verbindung gemäß den Ansprüchen 1 bis 11 zur Herstellung eines Arzneimittels mit nootropischer (neurotropischer) Wirkung.

**23.** Verwendung einer Verbindung gemäß den Ansprüchen 1 bis 11 zur Herstellung eines Arzneimittels zur Behandlung von cardiovaskulären Erkrankungen.

**24.** Verwendung einer Verbindung gemäß den Ansprüchen 1 bis 11 zur Herstellung eines Arzneimittels zur Behandlung von Haminkontinenz.

**25.** Verwendung einer Verbindung gemäß den Ansprüchen 1 bis 11 zur Herstellung eines Arzneimittels zur Behandlung von Diarrhöe.

**26.** Verwendung einer Verbindung gemäß den Ansprüchen 1 bis 11 zur Herstellung eines Arzneimittels zur Behandlung von Pruritus.

**27.** Verwendung einer Verbindung gemäß den Ansprüchen 1 bis 11 zur Herstellung eines Arzneimittels zur Behandlung von Entzündungen.

## Claims

**1.** 3-Amino-3-arylpropan-1-ol derivatives of the general formula I

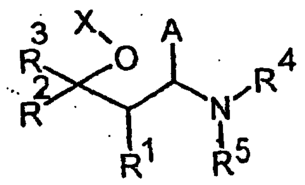

I

wherein

$R^1$, $R^2$ in each case independently of one another denote $C_{1-6}$-alkyl, or $R^1$ and $R^2$ together form a $(CH_2)_{2-6}$ ring, which can also be benzo-fused or phenyl- substituted,

$R^3$ denotes H or methyl,

$R^4$, $R^5$ in each case independently of one another denote $C_{1-6}$-alkyl, $C_3$-$C_6$-cycloalkyl, phenyl, benzyl, phenethyl, or $R^4$ and $R^5$ together form a $(CH_2)_{3-6}$ or $CH_2CH_2OCH_2CH_2$ ring,

A denotes a radical from the group consisting of substituted phenyl of the formula XIII

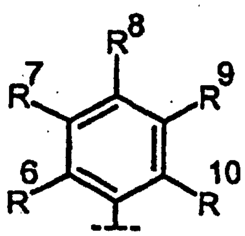

XIII

or represents a furanyl, thiophenyl, pyrrolyl, pyridinyl, pyrimidinyl, quinolinyl, isoquinolinyl, phthalazinyl or quinazolinyl radical which is mono- or polysubstituted by radicals chosen from the meanings given for $R^6$ to $R^{10}$,
wherein

$R^6$ to $R^{10}$ in each case independently of one another denote H, F, Cl, Br, I, $CF_3$, $OR^{11}$, $OCF_3$, $SR^{11}$, $SO_2CH_3$, $SO_2CF_3$, $C_{1-6}$-alkyl, phenyl, CN, $COOR^{11}$, $NO_2$, or $R^6$ and $R^7$ or $R^7$ and $R^8$ together form an $OCH_2O$ or $OCH_2CH_2O$ ring,

$R^{11}$ denotes $C_{1-6}$-alkyl, phenyl, benzyl, phenethyl,

X denotes a substituted benzyl of the formula XI

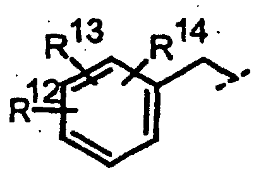

XI

or a substituted benzoyl of the formula XII

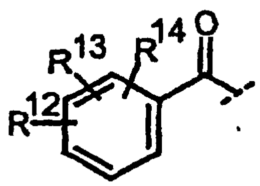

XII,

wherein

$R^{12}$ to $R^{14}$ in each case independently of one another denote H, F, Cl, Br, $CHF_2$, $CF_3$, $OR^{11}$, $SR^{11}$, $OCF_3$, $SO_2CH_3$, $SO_2CF_3$, $C_{1-6}$-alkyl, phenyl, CN, $COOR^{11}$, $NO_2$, where

$R^{11}$ denotes H, $C_{1-6}$-alkyl, phenyl, benzyl, phenethyl,

and diastereomers or enantiomers thereof, in the form of their bases or salts of physiologically acceptable acids.

2. Compounds according to claim 1, **characterized in that** $R^1$ and $R^2$ together form a $(CH_2)_{2-6}$ ring, which can be benzo-fused or phenyl-substituted, $R^3$ to $R^5$, A and X have the meaning according to claim 1.

3. Compounds according to claim 1, **characterized in that** $R^1$ and $R^2$ together form a $(CH_2)_4$ ring, which can be benzo-fused or phenyl-substituted, $R^3$ to $R^5$, A and X have the meaning according to claim 1.

4. Compounds according to claim 1, **characterized in that** $R^3$ represents hydrogen, $R^1$, $R^2$, $R^4$, $R^5$, A and X have the meaning according to claim 1.

5. Compounds according to claim 1, **characterized in that** $R^1$ and $R^2$ together form a $(CH_2)_{2-6}$ ring, which can be benzo-fused or phenyl-substituted, $R^3$ represents hydrogen, $R^4$, $R^5$, A and X have the meaning according to claim 1.

6. Compounds according to claim 1, **characterized in that** $R^1$ and $R^2$ together form a $(CH_2)_4$ ring, which can be benzo-fused or phenyl-substituted, A denotes a radical from the group consisting of substituted phenyl of the formula XIII or thiophenyl or furanyl, $R^3$ to $R^5$ and X have the meaning according to claim 1.

7. Compounds according to claim 1, **characterized in that** $R^1$ and $R^2$ together form a $(CH_2)_4$ ring, A denotes a radical from the group consisting of substituted phenyl of the formula XIII or thiophenyl or furanyl, $R^3$ to $R^5$ and X have the meaning according to the definition of claim 1.

**8.** Compounds according to claim 1, **characterized in that** $R^1$ and $R^2$ together form a $(CH_2)_4$ ring, A denotes thiophenyl, $R^3$ to $R^5$ and X have the meaning according to the definition of claim 1.

**9.** Compounds according to claim 1, **characterized in that** $R^1$ and $R^2$ together form a $(CH_2)_4$ ring, A denotes furanyl, $R^3$ to $R^5$ and X have the meaning according to the definition of claim 1.

**10.** Compounds according to claim 1, **characterized in that** X represents a radical from the group consisting of substituted benzyl of the formula XI, $R^1$ to $R^5$ and A have the meaning according to claim 1.

**11.** Compounds according to claim 1:

dimethyl-{[2-(2-methylbenzyloxy)cyclohexyl]phenylmethyl}amine and the corresponding hydrochloride

[2-(dimethylaminophenylmethyl)cyclohexyl] 4-trifluoromethylbenzoate and the corresponding hydrochloride

[2-(dimethylaminophenylmethyl)cyclohexyl] 4-methoxybenzoate and the corresponding hydrochloride

{[2-(2-chlorobenzyloxy)cyclohexyl]-(2-chlorophenyl)-methyl}dimethylamine and the corresponding hydrochloride

{[2-(3-fluorobenzyloxy)cyclohexyl]phenylmethyl}-dimethylamine and the corresponding hydrochloride

{[2-(4-fluorobenzyloxy)cyclohexyl]phenylmethyl}-dimethylamine and the corresponding hydrochloride.

**12.** Medicaments comprising as an active compound at least one compound according to claims 1 to 11.

**13.** Medicaments comprising as active compounds a mixture of the enantiomers of a compound according to claims 1 to 11, wherein the two enantiomers are not present in equimolar amounts, and optionally further active compounds.

**14.** Medicaments comprising as active compounds a mixture of the enantiomers of a compound according to claims 1 to 11, wherein one of the enantiomers has a relative content of between 5 and 45 per cent by weight of the enantiomer mixture, and optionally further active compounds.

**15.** Process for the preparation of a compound according to claims 1 to 11, **characterized in that** a Mannich base of the general formula II

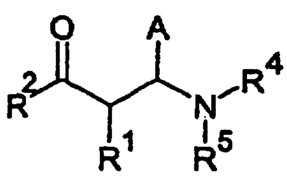

II,

wherein $R^1$ to $R^5$ and A have the meaning according to the general formula I, is reacted with a Grignard compound of the general formula $(H_3C)Y$, wherein Y denotes MgCl, MgBr or MgI, or MeLi or a reducing agent, preferably from the group consisting of sodium borohydride, sodium cyanoborohydride, lithium aluminium hydride, diisobutylaluminium hydride or a complex analogue of these compounds, to give an alcohol of the general formulae 1d

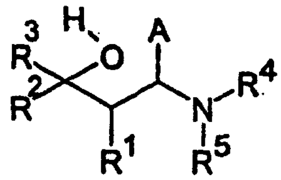

Id,

wherein
$R^1$ to $R^5$ and A have the meaning as in the general formula I,
an alcohol of the general formulae 1d is reacted with HalX, wherein Hal has the meaning of halogens from the group consisting of F, Cl, Br or I and X has the meaning according to the general formula I, in the presence of inorganic or organic bases in a temperature range of 0°-150°C or is subjected to a condensation reaction with XOH in a temperature range of 0°-150°C and is thus converted into a compound of the general formula I.

**16.** Use of a compound according to claims 1 to 11 for the preparation of a medicament for combating pain.

**17.** Use according to claim 16 for the preparation of a medicament for combating neuropathic pain.

**18.** Use according to claim 16 for the preparation of a medicament for combating chronic pain.

**19.** Use of a compound according to claims 1 to 11 for the preparation of a medicament with a local anaesthetic action.

**20.** Use of a compound according to claims 1 to 11 for the preparation of a medicament with an antiarrhythmic action.

**21.** Use of a compound according to claims 1 to 11 for the preparation of a medicament with an antiemetic action.

**22.** Use of a compound according to claims. 1 to 11 for the preparation of a medicament with a nootropic (neurotropic) action.

**23.** Use of a compound according to claims 1 to 11 for the preparation of a medicament for treatment of cardiovascular diseases.

**24.** Use of a compound according to claims 1 to 11 for the preparation of a medicament for treatment of urinary incontinence.

**25.** Use of a compound according to claims 1 to 11 for the preparation of a medicament for treatment of diarrhoea.

**26.** Use of a compound according to claims 1 to 11 for the preparation of a medicament for treatment of pruritus.

**27.** Use of a compound according to claims 1 to 11 for the preparation of a medicament for treatment of inflammations.

**Revendications**

**1.** Dérivés de 3-amino-3-arylpropane-1-ols de formule générale I

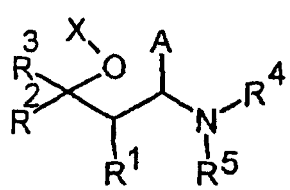

I

dans laquelle

$R^1$, $R^2$ représentent chacun, indépendamment l'un de l'autre, un reste alkyle en $C_1$ à $C_6$ ou bien $R^1$ et $R^2$ forment ensemble un noyau $(CH_2)_{2 \text{ à } 6}$, qui peut aussi être condensé au benzène ou substitué par un radical phényle,

$R^3$ représente H ou un reste méthyle,

$R^4$, $R^5$ représentent chacun, indépendamment l'un de l'autre, un reste alkyle en $C_1$ à $C_6$, cycloalkyle en $C_3$ à $C_6$, phényle, benzyle, phénéthyle ou bien $R^4$ et $R^5$ forment ensemble un noyau $(CH_2)_{3 \text{ à } 6}$ ou $CH_2CH_2OCH_2CH_2$,

A représente un reste choisi dans le groupe d'un reste phényle substitué de formule XIII

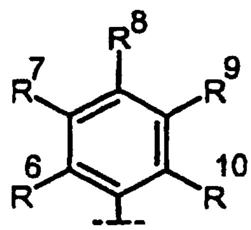

XIII

ou d'un reste furannyle, thiophényle, pyrrolyle, pyridinyle, pyrimidinyle, quinolinyle, isoquinolinyle, phtalazinyle ou quinazolinyle substitué une ou plusieurs fois par des restes qui sont choisis parmi les définitions indiquées pour les radicaux $R^6$ à $R^{10}$,

où

$R^6$ à $R^{10}$ représentent chacun, indépendamment l'un de l'autre, H, F, Cl, Br, I, $CF_3$, OH, $OR^{11}$, $OCF_3$, $SR^{11}$, $SO_2CH_3$, $SO_2CF_3$, alkyle en $C_1$ à $C_6$, phényle, CN, $COOR^{11}$, $NO_2$, ou bien $R^6$ et $R^7$ ou $R^7$ et $R^8$ forment ensemble un noyau $OCH_2O$ ou $OCH_2CH_2O$,

$R^{11}$ représente un reste alkyle en $C_1$ à $C_6$, phényle, benzyle, phénéthyle,

X désigne un reste benzyle substitué de formule XI

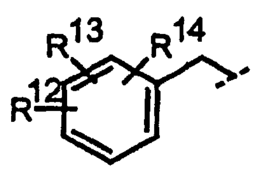

XI

ou un reste benzoyle substitué de formule XII

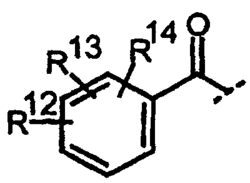

XII,

formules dans lesquelles

$R^{12}$ à $R^{14}$ représentent chacun, indépendamment l'un de l'autre, H, F, Cl, Br, $CHF_2$, $CF_3$, $OR^{11}$, $SR^{11}$, $OCF_3$, $SO_2CH_3$, $SO_2CF_3$, alkyle en $C_1$ à $C_6$, phényle, CN, $COOR^{11}$, $NO_2$,

$R^{11}$ représentant H, un radical alkyle en $C_1$ à $C_6$, phényle, benzyle, phénéthyle,

et leurs diastéréoisomères ou leurs énantiomères sous formes de leurs bases ou de leurs sels d'acides acceptables du point de vue physiologique.

2. Composés suivant la revendication 1, **caractérisés en ce que** $R^1$ et $R^2$ forment ensemble un noyau $(CH_2)_{2\ à\ 6}$, qui peut être condensé au benzène ou porter un substituant phényle, $R^3$ à $R^5$, A et X ont la définition indiquée selon la revendication 1.

3. Composés suivant la revendication 1, **caractérisés en ce que** $R^1$ et $R^2$ forment ensemble un noyau $(CH_2)_4$, qui peut être condensé au benzène ou porter un substituant phényle, $R^3$ à $R^5$, A et X ont la définition indiquée selon la revendication 1.

4. Composés suivant la revendication 1, **caractérisés en ce que** $R^3$ est l'hydrogène, $R^1$, $R^2$, $R^4$, $R^5$, A et X ont la définition indiquée selon la revendication 1.

5. Composés suivant la revendication 1, **caractérisés en ce que** $R^1$ et $R^2$ forment ensemble un noyau $(CH_2)_{2\ à\ 6}$ qui peut être condensé au benzène ou porter un substituant phényle, $R^3$ représente l'hydrogène, $R^4$, $R^5$, A et X ont la définition indiquée selon la revendication 1.

6. Composés suivant la revendication 1, **caractérisés en ce que** $R^1$ et $R^2$ forment ensemble un noyau $(CH_2)_4$ qui peut être condensé au benzène ou porter un substituant phényle, A représente un reste du groupe phényle substitué de formule XIII ou thiophényle ou furannyle, $R^3$ à $R^5$ et X ont la définition indiquée selon la revendication 1.

7. Composés suivant la revendication 1, **caractérisés en ce que** $R^1$ et $R^2$ forment ensemble un noyau $(CH_2)_4$, A est un reste du groupe phényle substitué de formule XIII ou thiophényle ou furannyle, $R^3$ à $R^5$ et X ont la signification selon la définition de la revendication 1.

8. Composés suivant la revendication 1, **caractérisés en ce que** $R^1$ et $R^2$ forment ensemble un noyau $(CH_2)_4$, A est un reste thiophényle, $R^3$ à $R^5$ et X ont la signification indiquée selon la définition de la revendication 1.

9. Composés suivant la revendication 1, **caractérisés en ce que** $R^1$ et $R^2$ forment ensemble un noyau $(CH_2)_4$, A est un reste furannyle, $R^3$ à $R^5$ et X ont la signification indiquée selon la définition de la revendication 1.

10. Composés suivant la revendication 1, **caractérisés en ce que** X est un reste du groupe d'un reste benzyle substitué de formule XI, $R^1$ à $R^5$ et A ont la signification indiquée selon la revendication 1.

11. Composés suivant la revendication 1, à savoir :

diméthyl-{[2-(2-méthylbenzyloxy)cyclohexyl]-phénylméthyl}amine et le chlorhydrate correspondant
[2-(diméthylaminophénylméthyl]cyclohexyl]-4-trifluorométhylbenzoate et le chlorhydrate correspondant
[2-(diméthylaminophénylméthyl]cyclohexyl]-4-méthoxybenzoate et le chlorhydrate correspondant
([2-(2-chlorobenzyloxy)cyclohexyl]-(2-chlorophényl)méthyl}diméthylamine et le chlorhydrate correspondant
{[2-(3-fluorobenzyloxy)cyclohexyl]phénylméthyl}-diméthylamine et le chlorhydrate correspondant
{[2-(4-fluorobenzyloxy)cyclohexyl]phénylméthyl}-diméthylamine et le chlorhydrate correspondant.

12. Médicament contenant comme substance active au moins un composé suivant les revendications 1 à 11.

13. Médicament contenant comme substances actives un mélange des énantiomères d'un composé suivant les revendications 1 à 11, les deux énantiomères n'étant pas présents en quantités équimolaires, et le cas échéant d'autres substances actives.

14. Médicament contenant comme substances actives un mélange des énantiomères d'un composé suivant les revendications 1 à 11, l'un des énantiomères étant en proportion relative entre 5 et 45 % en masse par rapport au mélange d'énantiomères, et le cas échéant d'autres substances actives.

**15.** Procédé de production d'un composé suivant les revendications 1 à 11, **caractérisé en ce qu'**on fait réagir une base de Mannich de formule générale II

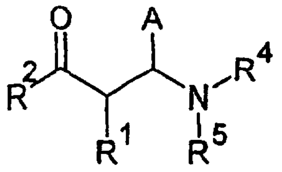

II

dans laquelle $R^1$ à $R^5$ et A ont la définition donnée pour la formule générale I, avec un composé de Grignard de formule générale $(H_3C)Y$, dans laquelle Y représente MgCl, MgBr ou MgI, ou bien MeLi ou un agent réducteur, de préférence du groupe du borohydrure de sodium, du cyanoborohydrure de sodium, de l'hydrure de lithium et d'aluminium, de l'hydrure de diisobutylaluminium ou d'un analogue complexe de ces composés, pour former un alcool de formule générale Id

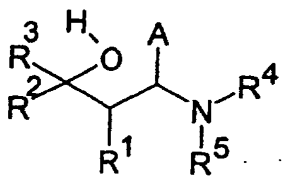

Id,

dans laquelle
$R^1$ à $R^5$ et A ont la définition donnée pour la formule générale I,

on fait réagir un alcool de formule générale Id avec HalX, où Hal représente des halogènes du groupe de F, Cl, Br ou I et X a la définition donnée selon la formule générale I, en présence de bases inorganiques ou organiques dans une plage de températures de 0 à 150°C, ou bien on le condense avec XOH dans une plage de températures de 0 à 150°C et on le transforme ainsi en un composé de formule générale I.

**16.** Utilisation d'un composé suivant les revendications 1 à 11 pour la préparation d'un médicament destiné à combattre la douleur.

**17.** Utilisation suivant la revendication 16, pour la préparation d'un médicament destiné à combattre des douleurs neuropathiques.

**18.** Utilisation suivant la revendication 16, pour la préparation d'un médicament destiné à combattre des douleurs chroniques.

**19.** Utilisation d'un composé suivant les revendications 1 à 11 pour la préparation d'un médicament destiné à une action anesthésique locale.

**20.** Utilisation d'un composé suivant les revendications 1 à 11 pour la préparation d'un médicament à action antiarythmique.

**21.** Utilisation d'un composé suivant les revendications 1 à 11 pour la préparation d'un médicament à action antiémétique.

**22.** Utilisation d'un composé suivant les revendications 1 à 11 pour la préparation d'un médicament à action nootropique (neurotropique).

**23.** Utilisation d'un composé suivant les revendications 1 à 11 pour la préparation d'un médicament destiné au traitement d'affections cardiovasculaires.

**24.** Utilisation d'un composé suivant les revendications 1 à 11 pour la préparation d'un médicament destiné au traitement de l'incontinence d'urine.

**25.** Utilisation d'un composé suivant les revendications 1 à 11 pour la préparation d'un médicament destiné au traitement de la diarrhée.

**26.** Utilisation d'un composé suivant les revendications 1 à 11 pour la préparation d'un médicament destiné au traitement du prurit.

**27.** Utilisation d'un composé suivant les revendications 1 à 11 pour la préparation d'un médicament destiné au traitement d'inflammations.